# EUROPEAN PATENT SPECIFICATION

(11) **EP 4 216 991 B1**
(45) Date of publication and mention of the grant of the patent: **25.11.2026**
(21) Application number: 21798853.4
(22) Date of filing: 23.09.2021
(51) Int. Cl.: C07K 14/47, A61K 38/00, A61P 35/00, A61K 39/00, G01N 33/575, C12Q 1/68

(54) **DISCOVERY AND USE OF IMMUNOGENIC PEPTIDES FOR THE TREATMENT AND PREVENTION OF CANCERS**
ENTDECKUNG UND VERWENDUNG IMMUNOGENER PEPTIDE ZUR BEHANDLUNG UND PRÄVENTION VON KREBS
DÉCOUVERTE ET UTILISATION DE PEPTIDES IMMUNOGÈNES POUR LE TRAITEMENT ET LA PRÉVENTION DE CANCERS

(30) Priority: 23.09.2020 US 202063082160 P
(43) Date of publication of application: 02.08.2023
(73) Proprietor: University of Houston System, Houston, Texas 77004 (US); Board of Regents of the University of Texas System, Austin, TX 78701 (US)
(72) Inventor: GUNARATNE, Preethi, Houston, Texas 77005 (US); MISTRETTA, Brandon, Houston, Texas 77204 (US); RANKOTHGEDERA, Sakuni, Houston, Texas 77004 (US); CASTILLO, Micah, Houston, Texas 77004 (US); BEDROSIAN, Isabelle, Bellaire, Texas 77401 (US)
(74) Representative: HGF
(86) International application number: PCT/US2021/051637
(87) International publication number: WO 2022/066828

(56) References cited:
- WO-A1-2017/177207
- "Paper 151", 1 January 2016, article LI YOU: "ChimeRScope: a novel alignment-free algorithm for fusion gene prediction using paired-end short reads", XP055867995

## Description

### CROSS-REFERENCE TO RELATED APPLICATIONS

This application claims priority to U.S. Provisional Patent Application No. 63/082,160, filed on September 23, 2020.

### STATEMENT REGARDING FEDERALLY FUNDED RESEARCH

This invention was made with government support under 1U01CA189240-01 awarded by the National Institutes of Health. The government has certain rights in the invention.

### BACKGROUND

Current methods of utilizing peptides derived from chimeric RNAs for treating or preventing cancer suffer from numerous limitations, such as limited applicability to different patients suffering from the same cancer. Numerous embodiments of the present disclosure address the aforementioned limitations.
WO2017/177207 (Bostongene LLC) describes a method of selecting a therapeutic fusion-specific vaccine for a cancer patient.
Li You "ChimeRScope: a novel alignemment-free alogrithm for fusion gene predicition using paired-end short reads", Paper 151, 1 January 2016, describes the use of ChimeRScope for the identification of chimeric fusion transcripts associated with cancer, amongst others the chimeric LRRC37A3&NSF fusion transcript.

### SUMMARY

The scope of the invention is defined by the claims. Any reference in the description to methods of treatment refer to the products of the present invention for use in a method for treatment. In one aspect the present invention provides a composition comprising: at least one immunogenic peptide, a nucleotide sequence that expresses the immunogenic peptide, or combinations thereof, wherein the immunogenic peptide comprises one or more peptides selected from the group consisting of ENDIKPKF (SEQ ID NO: 6), NDIKPKFP (SEQ ID NO: 7), ISNQNFQG (SEQ ID NO: 16), SNQNFQGN (SEQ ID NO: 17), analogs thereof, homologs thereof, or combinations thereof, wherein the analog or a homolog is at least 80% identical to any of the immunogenic peptides.

In some embodiments, the present disclosure pertains to methods of treating or preventing breast cancer in a subject. In some embodiments, the methods of the present disclosure include administering to the subject at least one immunogenic peptide, a nucleotide sequence that expresses the immunogenic peptide, or combinations thereof. Thereafter, the administered or expressed immunogenic peptide elicits an immune response against cells associated with the cancer.

In some embodiments, the immunogenic peptide is expressed by one or more chimeric nucleotide sequences derived from cells associated with the cancer. In some embodiments, the one or more chimeric nucleotide sequences have a higher prevalence in cancer cells when compared to non-cancer cells. In some embodiments, the immunogenic peptide includes a neoantigenic region. In some embodiments, the immunogenic peptide includes, without limitation, one or more of the following peptides: ENDIKPKF (SEQ ID NO: 6), NDIKPKFP (SEQ ID NO: 7), ISNQNFQG (SEQ ID NO: 16), SNQNFQGN (SEQ ID NO: 17), analogs thereof, homologs thereof, or combinations thereof.

Additional embodiments of the present disclosure pertain to compositions that include at least one immunogenic peptide of the present disclosure, a nucleotide sequence that expresses the immunogenic peptide, or combinations thereof.

### DESCRIPTION OF THE DRAWINGS

**FIG. 1A** illustrates a method of treating or preventing a cancer in a subject in accordance with numerous embodiments of the present disclosure.
**FIG. 1B** illustrates a method of identifying immunogenic peptides that elicit an immune response against cells associated with a cancer.
**FIG. 2** illustrates the fusion sequence of *N-ethylmaleimide sensitive factor, vesicle fusing ATPase, transcript variant 1 (NSF)* and *Leucine Rich Repeat Containing 37 Member A3 (LLRC37A3) (NSF-LRRC37A3,* NSF [Exon 1-12]ILRRC37A2[Exon 2-14]).
**FIG. 3** summarizes experimental results that validate the presence of NSF [Exon 1-12]ILRRC37A2 [Exon 2-14] fusion transcripts.
**FIG. 4** provides an illustration of the NSF-LRRC37A2 fusion transcript.
**FIG. 5** illustrates the selection of peptides from the NSF-LRRC37A2 fusion transcript and their immunogenicity validation. The peptides were selected through the MHC nuggets pipeline (Karchin Lab, John Hopkins University) based on their binding affinity (IC₅₀) to different classes of MHC alleles.
**FIG. 6** further illustrates the selected peptides from the NSF-LRRC37A2 fusion transcript and their immunogenicity validation.
**FIG. 7** illustrates a scheme for the further screening of immunogenic peptides for effectiveness as vaccine candidates.
**FIG. 8** illustrates images of the ELISpot plate with control wells and sample wells that were utilized to screen immunogenic peptides.
**FIG. 9** shows data related to a human IFNγ dual colour ELISpot Assay for predicted immunogenic neoantigenic peptides of NSF-LRRC37A2.
**FIG. 10** illustrates a synthetic mRNA vaccine design that can express immunogenic peptides.

### DETAILED DESCRIPTION

It is to be understood that both the foregoing general description and the following detailed description are illustrative and explanatory, and are not restrictive of the subject matter, as claimed. In this application, the use of the singular includes the plural, the word "a" or "an" means "at least one", and the use of "or" means "and/or", unless specifically stated otherwise. Furthermore, the use of the term "including", as well as other forms, such as "includes" and "included", is not limiting. Also, terms such as "element" or "component" encompass both elements or components comprising one unit and elements or components that include more than one unit unless specifically stated otherwise.

The section headings used herein are for organizational purposes and are not to be construed as limiting the subject matter described.

In the event that one or more of the incorporated literature and similar materials defines a term in a manner that contradicts the definition of that term in this application, this application controls.

Chimeric RNAs generated through chromosomal rearrangements (e.g., translocations, deletions, duplication and inversions), trans-splicing or read-through transcription provide optimal reagents for developing tumor vaccines. For instance, neoantigens generated from fusion transcripts have been reported to be better candidates for developing tumor vaccines because they are usually associated with significantly higher immunogenic potential than point mutation, single-nucleotide variants (SNV), or in-del based neoantigens.

The unique junctions formed in chimeric RNAs and the fusions proteins that are translated represent tumor-specific neoantigens. Neoantigens generated from novel proteins (i.e., from gene fusions) and/or truncated proteins (i.e., from 5'-gene and/or 3'-gene segments) are capable of inducing anti-tumor immune responses. Neoantigens can be exploited to design tumor vaccines and peptide-mediated T-cell activation to supplement both chemo and immunotherapies targeting cancer cells.

An inverse relationship has been reported between neoantigen recurrence and neoantigen immunogenicity. For instance, fusions involving cancer drivers (e.g., oncogenic genes and tumor passenger genes) were reported to have remarkably low immunogenic potentials, likely because they undergo selection pressure during tumorigenesis. By contrast, the largely private fusions present in 1-2 patients were highly immunogenic. Collectively, these findings suggest that vaccination-based cancer immunotherapy will be most successful when conducted through personalized strategies. However, a need exists for such vaccination-based methods to be more broadly applicable to different patients suffering from the same cancer. Numerous embodiments of the present disclosure address the aforementioned need.

In some embodiments, the present disclosure pertains to methods of treating or preventing breast cancer in a subject. In some embodiments, the methods of the present disclosure include: administering to the subject one or more immunogenic peptides, one or more nucleotide sequences that express the immunogenic peptide, or combinations thereof. In some embodiments, the immunogenic peptide is expressed by one or more chimeric nucleotide sequences derived from cells associated with the cancer. Thereafter, the administered or expressed immunogenic peptide elicits an immune response against cells associated with the cancer and results in the treatment or prevention of the cancer in the subject.

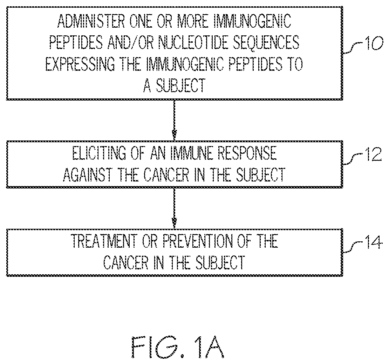

In more specific embodiments illustrated in **FIG. 1A****,** the methods of the present disclosure include: co-administering to the subject one or more immunogenic peptides and/or nucleotide sequences expressing the immunogenic peptides (step 10). In some embodiments, the administering can occur along with one or more immune adjuvants. Thereafter, the administered immunogenic peptide or the expressed immunogenic peptide from the administered nucleotide sequence elicits an immune response against cells associated with the cancer (e.g., cancer cells and/or precancerous cells) (step 12) and results in the treatment or prevention of the cancer in the subject (step 14). Additional embodiments of the present disclosure pertain to the immunogenic peptides of the present disclosure.

The description describes methods of identifying immunogenic peptides that elicit an immune response against cells associated with a cancer.

The description describes methods of screening cells associated with the cancer for one or more chimeric nucleotide sequences, identifying peptides expressed by the chimeric nucleotide sequences, and selecting immunogenic peptides from the identified peptides.

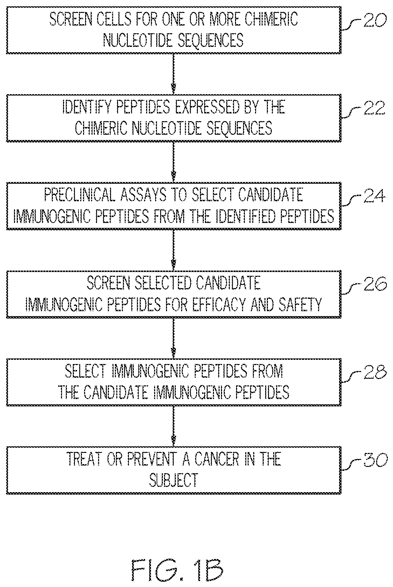

In more specific embodiments illustrated in **FIG. 1B****,** the methods described screening cells associated with the cancer (e.g., cancer cells or histologically normal appearing cells in cancer patients) for one or more chimeric nucleotide sequences (step 20), identifying peptides expressed by the chimeric nucleotide sequences (step 22), conducting a preclinical assay to select candidate immunogenic peptides from the identified peptides (step 24), screening selected candidate immunogenic peptides for efficacy and safety (step 26), and selecting the immunogenic peptides from the candidate immunogenic peptides (step 28).

The selected immunogenic peptides are then utilized to treat or prevent a cancer in a subject (e.g., step 30 in **FIG. 1B**). The treatment or prevention of the cancer occurs in accordance with the methods of the present disclosure.

The description describes various methods may be utilized to screen, identify, and select immunogenic peptides that elicit immune responses against various cancers. Moreover, various methods may be utilized to administer various immunogenic peptides that are expressed by various cancer-derived chimeric nucleotide sequences in order to elicit various immune responses against various cancers.

### Screening of cancer cells for chimeric nucleotide sequences

The methods described herein may screen cancer cells for various types of chimeric nucleotide sequences. For instance, The chimeric nucleotide sequence is in the form of a DNA sequence , or chimeric nucleotide sequence is in the form of an RNA sequence, or the chimeric nucleotide sequence is in the form of a messenger RNA (mRNA) sequence.

Various methods may be utilized to screen cancer cells for chimeric nucleotide sequences. For instance, the screening occurs by nucleotide sequencing to identify chimeric nucleotide sequences. The nucleotide sequencing includes RNA sequencing. The identified chimeric nucleotide sequences are compared against non-cancer cell sequences to identify chimeric nucleotide sequences that are recurrent in cancer cells.

### Identification of peptides expressed by the chimeric nucleotide sequences

Various methods may also be utilized to identify peptides expressed by the chimeric nucleotide sequences. For instance, the identifying includes deciphering the peptide sequences from the one or more chimeric nucleotide sequences.
An algorithm may be utilized to decipher the peptide sequences from the one or more chimeric nucleotide sequences.

### Selecting immunogenic peptides from the identified peptides

Various methods may also be utilized to select immunogenic peptides from the identified peptides. For instance, the selecting includes predicting the ability of the identified peptides to elicit an immune response against cells associated with a cancer.

The predicting includes predicting the ability of the peptide to bind to human leukocyte antigen (HLA) systems or complexes. The HLA systems or complexes include major histocompatibility complex (MHC) proteins, MHC class I (MHC I) proteins, MHC class II (MHC II) proteins, or combinations thereof.

The predicting occurs by utilizing an algorithm.

The algorithm includes a neural network that predicts the ability of the peptide to bind to human leukocyte antigen (HLA) systems or complexes.

The selecting includes testing the ability of the peptide to bind to human leukocyte antigen (HLA) systems or complexes (e.g., MHC proteins, MHC I proteins, MHC II proteins, or combinations thereof). The testing occurs through the utilization of an assay.

The selection of immunogenic peptides from the identified peptides includes: (a) conducting a preclinical assay to select candidate immunogenic peptides from the identified peptides; (b) screening selected candidate immunogenic peptides for efficacy and safety; and (c) selecting the immunogenic peptides from the candidate immunogenic peptides.

### Chimeric nucleotide sequences

Chimeric nucleotide sequences generally refer to nucleotide sequences that contain exons from one or more genes, and that express the immunogenic peptides of the present disclosure. In some embodiments, the chimeric nucleotide sequences of the present disclosure contain exons from two or more genes.

In some embodiments, the chimeric nucleotide sequences of the present disclosure have a higher prevalence in cancer cells when compared to non-cancer cells. In some embodiments, such a higher prevalence is determined through comparison of copy numbers from RNA-sequencing data obtained from cancer cells and control samples from normal tissue (e.g., normal breast tissue).

In some embodiments, the chimeric nucleotide sequences of the present disclosure include chimeric DNA sequences. In some embodiments, the chimeric nucleotide sequences of the present disclosure include chimeric RNA sequences. In some embodiments, the chimeric RNA sequences are supported by underlying DNA changes. In some embodiments, the underlying DNA changes include, without limitation, deletions, duplications, insertions, translocations, inversions, or combinations thereof. In some embodiments, the altered DNA generates during transcription the chimeric RNAs with the potential to be translated into fusion proteins and harbor neoantigen sites within immunogenic neopeptides. In some embodiments, the underlying DNA changes bring together two genes that during translation can give rise to form in-frame fusions and/or 5' and 3'-truncations.

In some embodiments, the chimeric nucleotide sequences include chimeric RNAs without accompanying DNA changes. For instance, in some embodiments, the chimeric RNAs are the products of transplicing events without accompanying DNA changes. In some embodiments, the chimeric nucleotide sequences of the present disclosure include a junction point with one end that maps on one gene and another end that maps on another gene. In some embodiments, the immunogenic peptides of the present disclosure include peptide sequences that are expressed at such junction points of the chimeric nucleotide sequences to form polypeptides of a single protein (e.g., a truncated protein) or two proteins (e.g., fusion proteins).

In some embodiments, the junction point is at a junction region between *N-ethylmaleimide sensitive factor, vesicle fusing ATPase, transcript variant 1 (NSF)* and *Leucine Rich Repeat Containing 37 Member A3 (LLRC37A3) (NSF-LRRC37A3).* In some embodiments, the junction region includes a sequence of CTGCAAGTGATGAGAGGAGACTTCCTTGCTTCTTTGGAGAATGATATCAAACCA AAATTTCCAAGGAAACTATATTGAAAATAACTTGACTGAATTACACAAGGATTCATT TGAAGGCCTGCTATCCCTCCAGTATTTAGATTTATCCTGCG (SEQ ID NO: 21).

### Cancer-associated cells

The chimeric nucleotide sequences of the present disclosure can be derived from various cancer-associated cells. For instance, in some embodiments, the cells associated with the cancer include, without limitation, cancer cells, normal cells, precancerous cells, precancerous lesions, precancerous tumors, cancerous lesions, cancerous tumors, cells near cancerous lesions, cells near cancerous tumors, histologically normal appearing cells in subjects suffering from a cancer (e.g., histologically normal areas adjacent to tumor), or combinations thereof.

In some embodiments, the cells associated with the cancer include cancer cells and cells near the cancer cells. In some embodiments, the cells near the cancer cells include at least one of precancerous cells, non-cancerous cells, or combinations thereof. In some embodiments, the cells near the cancer cells are in the form of a non-cancerous or pre-cancerous tissue that is adjacent to or near a cancerous tissue. In some embodiments, the cancerous tissue contains the cancer cells.

In some embodiments, the non-cancerous or pre-cancerous tissue is within less than 10 cm of the cancerous tissue. In some embodiments, the non-cancerous or pre-cancerous tissue is within less than 20 cm of a cancerous tissue. In some embodiments, the non-cancerous or pre-cancerous tissue is within less than 50 cm of a cancerous tissue. In some embodiments, the non-cancerous or pre-cancerous tissue is within less than 100 cm of a cancerous tissue. In some embodiments, the non-cancerous or pre-cancerous tissue is within less than 250 cm of a cancerous tissue. In some embodiments, the non-cancerous or pre-cancerous tissue is located within the same organ that contains the cancerous tissue.

In more specific embodiments, the cells associated with the cancer include cells near histologically normal areas throughout the breast of a breast cancer patient, including the contralateral unaffected breast, which represents a molecular field alteration throughout the breasts of patients diagnosed with breast cancer.

### Administration of immunogenic peptides to subjects

The immunogenic peptides of the present disclosure and nucleotides expressing the immunogenic peptides may be administered to subjects through various administration routes. For instance, in some embodiments, the administration routes include, without limitation, oral administration, inhalation, subcutaneous administration, intravenous administration, intraperitoneal administration, intramuscular administration, intrathecal injection, intra-articular administration, topical administration, central administration, peripheral administration, aerosol-based administration, nasal administration, transmucosal administration, transdermal administration, parenteral administration, and combinations thereof. In some embodiments, the administration occurs by intravenous administration.

The immunogenic peptides of the present disclosure and nucleotides expressing the immunogenic peptides can be administered in various forms. For instance, in some embodiments, the immunogenic peptides of the present disclosure and nucleotides expressing the immunogenic peptides are co-administered with one or more immune adjuvants. In some embodiments, the immunogenic peptides of the present disclosure and nucleotides expressing the immunogenic peptides are administered in the form of a peptide vaccine.

In some embodiments, the administering includes administering an immunogenic peptide of the present disclosure. In some embodiments, the administering includes administering a nucleotide sequence expressing an immunogenic peptide of the present disclosure. In some embodiments, the nucleotide sequence is in the form of a DNA sequence. In some embodiments, the nucleotide sequence is in the form of an RNA sequence. In some embodiments, the nucleotide sequence is in the form of a mRNA sequence.

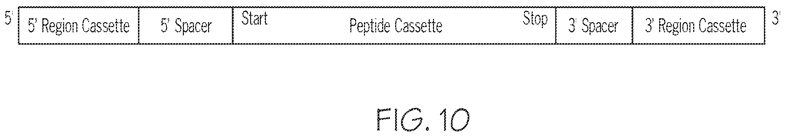

In some embodiments, the nucleotide sequence includes a mRNA expression cassette. In some embodiments, the mRNA expression cassette includes a DNA sequence, such as a doublestranded DNA sequence. In some embodiments, the mRNA expression cassette includes a mRNA sequence. In some embodiments illustrated in **FIG. 10****,** the mRNA expression cassette includes a peptide cassette that contains the nucelotide sequence expressing the immunogenic peptide, a 5' cassette region upstream the peptide cassette, a spacer region between the 5' cassette region and the peptide cassette, a 3' cassette region downstream the peptide cassette, and a spacer region between the peptide cassette and the 3' cassette region. In some embodiments, at least one of the 5' cassette region and 3' cassette region is designed to optimize the expression of the immunogenic peptide. In some embodiments, the 5' cassette region is designed to optimize the expression of the immunogenic peptide. In some embodiments, the 3' cassette region is designed to optimize the expression of the immunogenic peptide. In some embodiments, the 3' and 5' cassette regions are both designed to optimize the expression of the immunogenic peptide.

### Eliciting of an immune response against cells associated with a cancer

The immunogenic peptides of the present disclosure can elicit various immune responses in a subject against cells associated with a cancer. For instance, in some embodiments, the immunogenic peptides of the present disclosure elicit or are capable of eliciting an immune response through binding to human leukocyte antigen (HLA) systems or complexes. In some embodiments, the HLA systems or complexes include, without limitation, major histocompatibility complex (MHC) proteins, MHC class I (MHC I) proteins, MHC class II (MHC II) proteins, or combinations thereof.

### Treatment or prevention of cancers

The methods of the present disclosure can be utilized to treat or prevent breast cancer in various subjects. In some embodiments, the methods of the present disclosure are utilized to treat a cancer. In some embodiments, the methods of the present disclosure are utilized to prevent a cancer. In some embodiments, the methods of the present disclosure are utilized to treat and prevent a cancer.

In some embodiments, the cancer to be treated or prevented includes a cancer with a low prevalence of mutations. In some embodiments, the low prevalence of mutations is determined through quantitative PCR (qPCR) using primer sets to the fusion junction of specific chimeric nucleotides.

In some embodiments, the cancer to be treated or prevented is breast cancer.

The methods of the present disclosure can be utilized to treat or prevent cancer in various subjects. For instance, in some embodiments, the subject is a human being. In some embodiments, the subject is suffering from a cancer to be treated or prevented. In some embodiments, the subject is vulnerable to the cancer. In some embodiments, the subject is vulnerable to the cancer through genetic susceptibility. In some embodiments, the subject is vulnerable to the cancer through environmental susceptibility.

### Immunogenic peptide compositions

The methods of the present disclosure can utilize various types of immunogenic peptides and nucleotide sequences that express the immunogenic peptides. Generally, immunogenic peptides refer to peptides that are capable of eliciting an immune response against cells associated with a cancer (e.g., cancerous and/or precancerous cells). Additional embodiments of the present disclosure pertain to compositions that include at least one immunogenic peptide, a nucleotide sequence that expresses the immunogenic peptide, or combinations thereof.

In some embodiments, the compositions of the present disclosure include at least one immunogenic peptide of the present disclosure. In some embodiments, the compositions of the present disclosure include a nucleotide sequence that expresses an immunogenic peptide of the present disclosure. In some embodiments, the nucleotide sequence is in the form of a DNA sequence. In some embodiments, the nucleotide sequence is in the form of an RNA sequence. In some embodiments, the nucleotide sequence is in the form of a mRNA sequence. In some embodiments, the nucleotide sequence is in the form of a mRNA expression cassette described herein.

In some embodiments, the immunogenic peptides of the present disclosure and nucleotide breast cancer sequences that express them are suitable for use in treating or preventing in a subject .

In some embodiments, the immunogenic peptides of the present disclosure include one or more neoantigenic regions. In some embodiments, the neoantigenic regions include amino acid sequences that had not been previously recognized by the immune system of a subject. In some embodiments, the neoantigenic regions of the present disclosure are not capable of eliciting an immune response against normal cells or tissues. In some embodiments, the immunogenic peptides of the present disclosure have no recognizable target in normal cells (e.g., non-cancerous cells). In some embodiments, the immunogenic peptides of the present disclosure represent moderately recurrent peptides that have escaped immune surveillance and have high promiscuity for binding a large pool of HLAs.

In some embodiments, the immunogenic peptides of the present disclosure include a polypeptide sequence of a single protein. In some embodiments, the single protein is a truncated protein. In some embodiments, the immunogenic peptides of the present disclosure include polypeptide sequences of two proteins, such as a fusion protein.

In some embodiments, the immunogenic peptides of the present disclosure include one or more peptides that include, without limitation,
ENDIKPKF (SEQ ID NO: 6), NDIKPKFP (SEQ ID NO: 7),
ISNQNFQG (SEQ ID NO: 16), SNQNFQGN (SEQ ID NO: 17),
analogs thereof, homologs thereof, or combinations thereof. or combinations thereof. In some embodiments, the immunogenic peptides of the present disclosure include one or more peptides that include, without limitation, ENDIKPKF (SEQ ID NO: 6), NDIKPKFP (SEQ ID NO: 7), ISNQNFQG (SEQ ID NO: 16), SNQNFQGN (SEQ ID NO: 17),
analogs thereof, homologs thereof, or combinations thereof.

In some embodiments, the analog is at least 80% identical to any of the immunogenic peptides of the present disclosure. In some embodiments, the analog is at least 85% identical to any of the immunogenic peptides of the present disclosure. In some embodiments, the analog is at least 90% identical to any of the immunogenic peptides of the present disclosure. In some embodiments, the analog is at least 95% identical to any of the immunogenic peptides of the present disclosure.

In some embodiments, the homolog is at least 80% identical to any of the immunogenic peptides of the present disclosure. In some embodiments, the homolog is at least 85% identical to any of the immunogenic peptides of the present disclosure. In some embodiments, the homolog is at least 90% identical to any of the immunogenic peptides of the present disclosure. In some embodiments, the homolog is at least 95% identical to any of the immunogenic peptides of the present disclosure.

The immunogenic peptides of the present disclosure and nucleotide sequences that express them can be embedded in various additional components. For instance, in some embodiments, the immunogenic peptides of the present disclosure and nucleotide sequences that express them can be embedded in a pharmaceutical composition. In some embodiments, the pharmaceutical composition can include, without limitation, solubilizing agents, pharmaceutically acceptable carriers, excipients, syrups, elixir, water, gels, and combination thereof.

In some embodiments, the immunogenic peptides of the present disclosure and nucleotide sequences that express them can be in a composition that also includes one or more immune adjuvants. In some embodiments, the one or more immune adjuvants include, without limitation, analgesic adjuvants, inorganic compounds, mineral oil, bacterial products, non-bacterial inorganics, delivery systems, plant-based products, cytokines, food-based oil, or combinations thereof.

The immunogenic peptides of the present disclosure and nucleotide sequences that express them can also be in various forms. For instance, in some embodiments, the immunogenic peptides of the present disclosure can be in the form of a peptide vaccine.

### Advantages

The embodiments of the present disclosure have numerous advantages. To begin with, most fusion transcript detection methods focus on paired-end sequence reads showing discordant mapping. On the other hand, various embodiments of the present disclosure focus on single reads that have to be broken up (i.e., junction crossing reads) because the 5'-end maps on one gene and the 3'-endmaps on another gene. Such an approach eliminates the requirement for paired-end reads and substantially increases the signal for fusion transcripts discovered through aberrant trans-splicing events during disease progression.

Moreover, much of the work on neoantigens in breasts and other cancers relates to single nucleotide variants (SNV) and small insertions and deletions (indel) within a single gene. On the other, various embodiments of the present disclosure focus on discovering neoantigens from fusion transcripts from two separate genes.

Additionally, various embodiments of the present disclosure are highly valuable to cancers with low mutational burdens. In particular, cancers that have low mutational burdens, such as breast cancer, provide limited opportunities for peptide vaccine development. Accordingly, the chimeric nucleotides that have been uncovered in various embodiments of the present disclosure, and the relatively large number of associated immunogenic peptides, opens the door for more cancer vaccines in tumors with relatively fewer somatic mutations.

Furthermore, vaccines for cancer prevention have a very high bar for selection of agents with little or no side-effects. On the other hand, the unique sequences at fusion junctions of the chimeric nucleotides of the present disclosure form new open reading frames (ORFs) from fusion proteins that represent a hybrid of the two founding genes and/or truncated versions of the two wild type proteins. This is due to premature termination of the 5'-gene yielding a unique amino acid sequence in the C- terminus and novel N-terminal region in the 3'-gene. Such immunogenic regions discovered and presented in the present disclosure will have no recognizable target in normal cells and therefore are expected to have little or no side effects.

Moreover, the immunogenic peptides of the present disclosure are potentially applicable to the prevention and treatment of numerous types of cancers. For instance, the pipeline for discovery of immunogenic peptides from RNA fusions developed in the present disclosure is applicable to providing reagents for developing tumor vaccines for the prevention and treatment of ovarian, lung, osteosarcoma and numerous types of other cancers.

Additionally, the methods of the present disclosure can be used to prioritize moderately recurrent fusions to select for immunogenicity and HLA-binding promiscuity for broad applicability to groups of patients. For instance, fusions involving cancer drivers (e.g., oncogenic genes and tumor passenger genes) were reported to have remarkably low immunogenic potentials, likely because they undergo selection pressure during tumorigenesis. By contrast, private fusions presented in 1-2 patients were highly immunogenic. Collectively, these findings suggest that vaccination-based cancer immunotherapy will be most successful when conducted through personalized strategies. On the other hand, in some embodiments, the immunogenic peptides of the present disclosure represent moderately recurrent fusions that have escaped immune surveillance and have high promiscuity for binding a large pool of HLAs, which are hypothesized to be broadly applicable to more patients than the private fusions.

Moreover, the selection of immunogenic peptides (e.g., fusions) that are present in early stages of cancer (e.g., adjacent normal and distant normal) are not necessarily selected to be sustained in a tumor. Fusions involving cancer drivers (e.g., oncogenic genes and tumor passenger genes) have remarkably low immunogenic potentials, likely because they are selected to evade the immune system. However, private fusions present in 1-2 patients are highly immunogenic. On the other hand, in some embodiments, the immunogenic peptides of the present disclosure represent moderately recurrent fusions that are expected to be moderately immunogenic (e.g., enough to escape immune surveillance) and at the same time be found across a larger pool of patients to form the basis for tumor vaccines that are broadly applicable.

### Additional Embodiments

Reference will now be made to more specific embodiments of the present disclosure and experimental results that provide support for such embodiments. However, Applicants note that the disclosure below is for illustrative purposes only and is not intended to limit the scope of the claimed subject matter in any way.

### Example 1. Chimeric RNAs Reveal Putative Neoantigen Peptides for Developing Tumor Vaccines for Breast Cancer

Unique amino acid sequences at the junctions of fusion or truncated proteins translated from chimeric RNAs form neoantigen peptide sites capable of inducing anti-tumor immune responses. In this Example, Applicant's objective was to find recurrent fusion transcripts that have the potential to generate candidate neoantigens presented by major histocompatibility complex class I (MHC I) during breast cancer progression.

Applicant comprehensively characterized the landscape of fusion transcripts in 225 samples of breast tumors representing 3 subtypes. For each patient, Applicant tested four sites, including Tumor (T), Adjacent Normal (Adj-NL), and Distant Normal (Dist-NL-2 sites). Using breast tissue from unaffected individuals (NL), Applicant uncovered 20 novel fusion transcript variants detected from RNAseq data analyzed through two fusion callers.

NSF-LRRC37A3, the fusion transcript with the largest number of junction crossing reads per sample and the highest recurrence, was selected for further study. NSF (N-ethylmaleimide sensitive factor, vesicle fusing ATPase, transcript variant 1) and LLRC37A3 (Leucine Rich Repeat Containing 37 Member A3) are located in 17q21.31 and 17q24.1 respectively. The fusion was detected in ~20% of the 75 tumor samples (TNBC=4/25, HER2+=7/25 and HR+=7/25), 5 samples in the TCGA breast cancer dataset and absent in NL (n=4).

Some fraction of the patients that presented NSF-LRRC37A3 in the tumor also contained the fusion in Adj-NL and/or Dist-NL. Interestingly, some patients who were fusion negative for the tumor scored fusion positive for the matched Adj-NL and Distant-NL.

The 5'- and 3'-boundaries were found located on the coding strands of Exon 12 of NSF and Exon 2 of LRRC37A3. The two major open reading frames (ORFs) were predicted, including NSF-Exon 1-12-KFPRKLYFLH (NSF with a C-terminal truncation) and MISNQN-LRRC37A3 Exon 2-14 (LRRC37A3 with an N-terminal truncation).

The two ORFs were analyzed through MHCnuggets, a deep neural network method that predicts peptide-MHC binding to MHC class I/II. A total of 18 different 8-11 mer neoantigen peptides discovered from the fusion ORFs were predicted to bind to a total of 30 unique MHC class I alleles with a binding affinity of IC50 < 500nM.

Applicant focused on extracting neoantigens from fusion transcripts from two separate genes. The unique sequences at the fusion junctions form new open reading frames (ORFs) that can result in 1) fusion proteins representing a hybrid of the two founding genes and/or 2) truncated versions of the two wild type proteins due to premature termination of the 5'-gene yielding a unique amino acid sequence in the C-terminus and novel N-terminal region in the 3'gene. Applicant's main objective was to discover immunogenic neoantigens that can be processed and presented by the major histocompatibility complex (MHC) Class I peptides during breast cancer progression to target CD8+ T cells. The ultimate goal in this Example is to extract immunogenic neopeptide regions that can form the basis for development of tumor vaccines for both treatment and prevention of breast cancer.

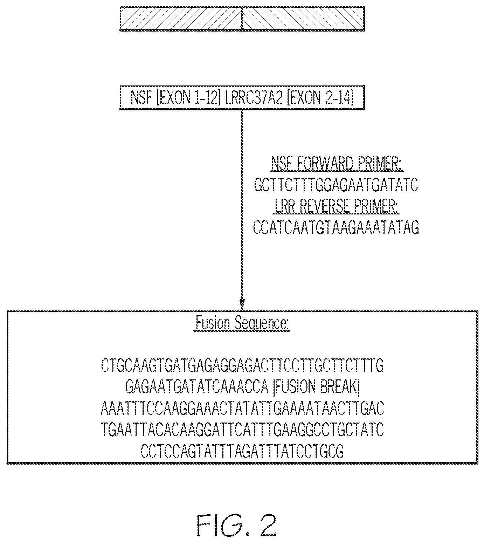

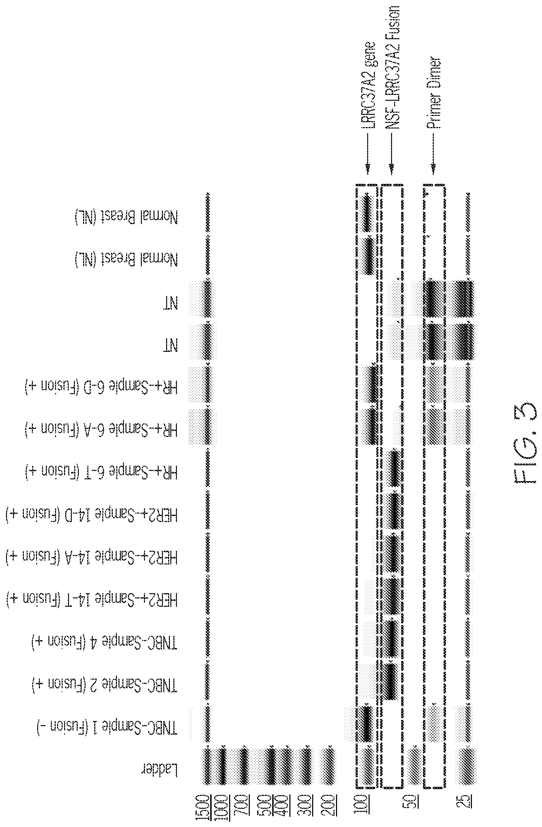

With the goal of discovering RNA-fusions that can be mined for neoantigen peptide candidates, Applicant performed RNA-Sequencing of triple negative (TNBC), Her2+ and hormone receptor positive (HR+) breast cancer samples (n=25 each) and compared with normal breast tissue samples (n=4). Using a split-read (junction crossing reads) and discordant read (junction spanning reads) mapping approach to detect chimeric RNAs, Applicant discovered 20 recurrent chimeric RNAs from Breast Cancer. The 20 fusion transcripts were detected in 1 or more samples from the TCGA dataset and absent in Normal Breast tissue (n=4). Of the 20 novel fusions found, the NSF-LRRC7A3 fusion transcript (**FIG. 2**) was selected for further study based on the fact that it was associated with the highest number of junction crossing reads (TNBC=218, HER2+=274, HR+=217) and was the most recurrent (TNBC=4 samples, Her2+=7 samples and HR+=7 samples, TCGA Breast Cancer Dataset= 5 samples from two independent clinical sites (University of Chicago = 4, MD Anderson cancer Center = 1)) (**FIG. 3**). Furthermore, the same exon boundary of NSF Exon 12|LRRC37A3 Exon 2 was identified by 2 different fusion callers including the CLC Genomics workbench 20.0 (Qiagen) and Xiaoping pipeline.

In order to characterize the immunogenic repertoire of the precancerous states during cancer progression, Applicant evaluated samples from Tumor (T), Adjacent Normal (Adj-NL) site adjacent to the tumor, and from a distant site on the affected breast (Dist-NL). Fusion transcripts identified from these samples were compared with normal breast tissue from unaffected individuals (NL) to remove false positives.

Of the 4 TNBC patients that presented NSF-LRRC37A3 in the tumor, 3 also contained the fusion in Adj-NL and 2 that did not contain the fusion in the tumor were shown to carry the fusion in the matched Adj-NL or Dist-NL. Similar patterns were observed in HER2⁺ and HR⁺. The results are summarized in **Table 1.** Of the 7 HER2⁺ patients and 7 HR⁺ that presented NSF-LRRC37A3 in the tumor, 3 also contained the fusion in Adj-NL of each subtype respectively. 3 HER2⁺ and 2 HR⁺ contained fusion in Dist-NL samples. Some patients who tested negative for the fusion in the tumor contained the fusion exclusively in Adj-NL or Dist-NL.

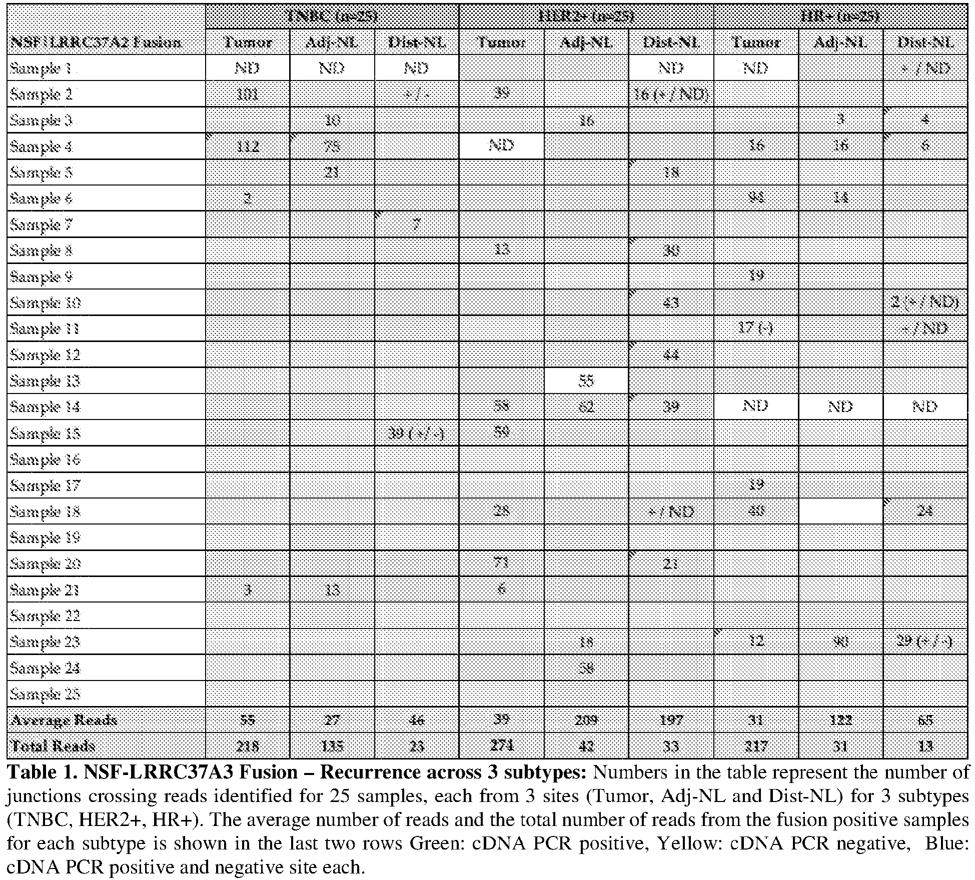

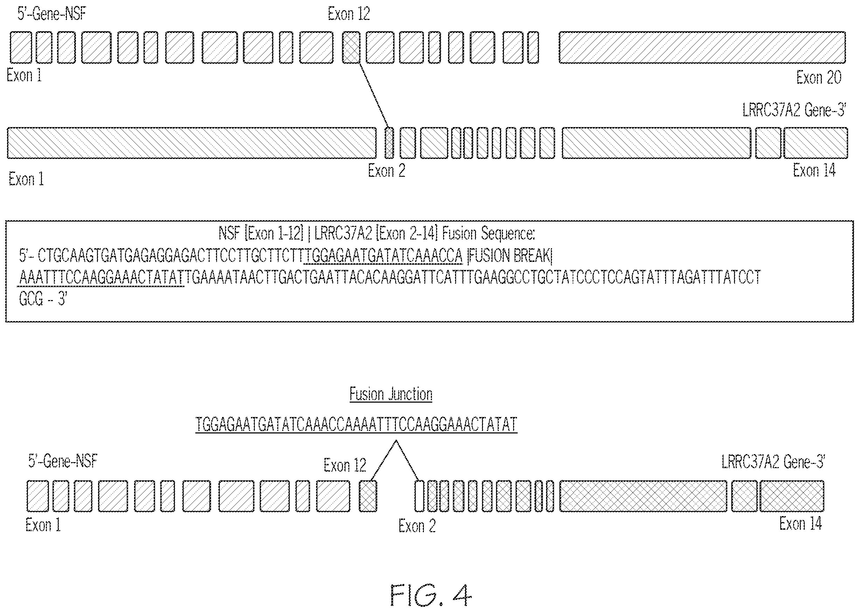

As illustrated in **FIG. 4****,** Applicant discovered that exon boundaries of the NSF-LRRC37A3 Fusion maps to Exon 12 of NSF and Exon 2 of LRR37CA3. To compile the NSF-LRRC37A3 fusion junction, Applicant extracted the sequence reads that had discordant mapping of the paired-end reads from RNAseq and the junction crossing reads.

To identify the exon boundaries of the fusions transcripts, Applicant mapped the complete set of unique junction crossing reads from each sub types on hg38 Refseq GRCh38.p9. The 5'-boundary of NSF-LRRC37A3 was found to be located on Exon 12 of NSF (NM_006178.4) and Exon 2 (NM_199340.4) of LRRC37A3 on the coding strand of both genes. The boundaries were consistent and supported by 986 junction-crossing reads (TNBC=218, HER2+=274 and HR+=217) with the breakpoint sequence always AAACCA on the NSF gene and AAATTC on LRRC37A3. The fusion junction and the exon boundaries model for the NSF-LRRC37A3 fusion are also shown in **FIG. 4****.**

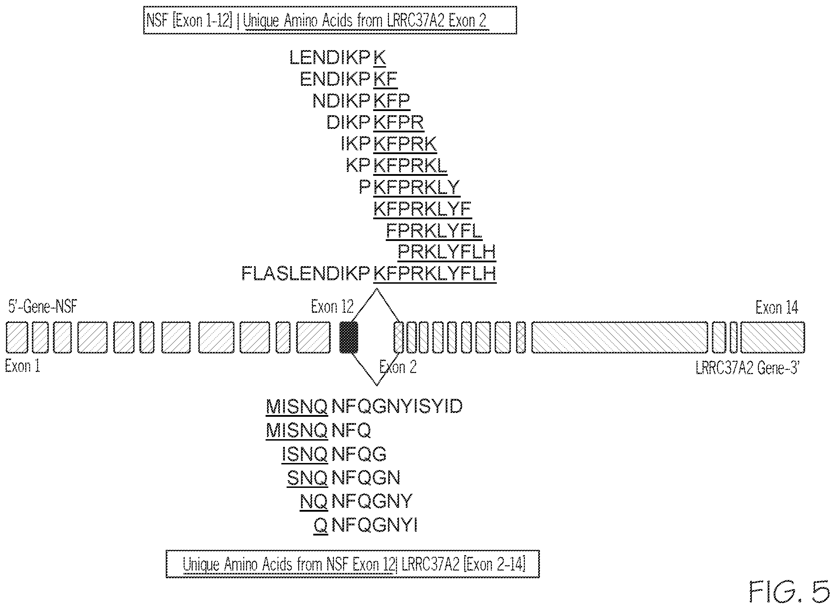

Applicant also discovered that novel fusion junctions from the NSF-LRRC37A3 fusion transcript variants contain two major ORFs generating two truncated proteins. The sequences of the ORFs predicted from the NSF [Exon 1-12]ILRRC37A3 [Exon 2-14] fusion are shown in **FIG. 5****.** Two regions of unique amino acid residues carrying neopeptides were uncovered from this analysis. Neopeptide regions were delineated from the 2 major ORFs predicted from the NSF [Exon 1-12]|LRRC37A3 [Exon 2-14] fusion. The truncated NSF protein yielded the unique peptide fragment KFPRKLYFLH at the C-terminal end of NSF Exons 1-12 and unique amino acids contributed by Exon 2 of LRRC37A3. The truncated LRRC37A3 protein yielded the unique peptide fragment MISNQN at the N-terminal end of LRRC37A3 Exons 2-14 and unique amino acids contributed by Exon 12 of NSF.

To assess the immunogenicity of the predicted neoantigens, a total 18 peptides of 8-11 amino acids extracted from the 2 major ORFs generated from the NSF-LRRC37A3 fusion were processed through the neoantigen prediction platform, MHCnuggets, which evaluates binding of somatic peptides to MHC class I, antigen processing, self-similarity and gene expression. A total of 106 HLA genotypes from Human served as input to MHCnuggets to predict the MHC class I binding potential (IC₅₀ nM) of each peptide region. Neoantigen candidates meeting an IC₅₀ affinity < 5000nM were subsequently ranked based on MHC binding. Anchor and auxiliary anchor residues for neopeptide-HLA class I allele pairs were evaluated by the SYFPEITHI online tool.

The peptides were then rank ordered for binding affinity to the most number of MHC class I alleles (promiscuity), antigen processing, and self-similarity. To identify the most promiscuous peptides, which have been shown to be strong vaccine candidates, Applicant ranked the peptides by number of HLA Class I allele that each peptide bound to at a binding affinity threshold of IC50 <500nM. While many of the peptides bind to less than 10 MHC class 1 alleles, a small fraction do bind to >20 MHC alleles, which were further investigated.

Applicant uncovered 12 and 6 immunogenic neoantigen peptides from the truncated NFS protein variant and the truncated LRRC37A3 protein variant, respectively. Applicant found 18 neoantigen peptides predicted to be presented by 1-6 MHC class I alleles with a binding affinity of IC50<50nM and 1-15 MHC class I alleles with a binding affinity of IC50<500nM.

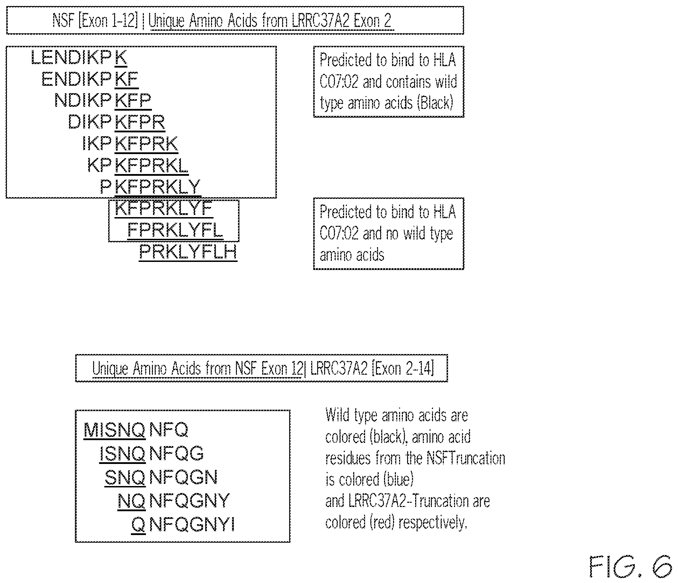

Previous studies have reported that predicted antigen with IC50<50 nM bind to strongly and do not initiate an immune response. Accordingly, Applicant chose to highlight MHC class I alleles with a binding affinity of IC50<500nM. These immunogenic neopeptides were found to bind to a total of 30 unique MHC Class I HLA types. The selected peptides are listed and illustrated in **FIG. 6****.**

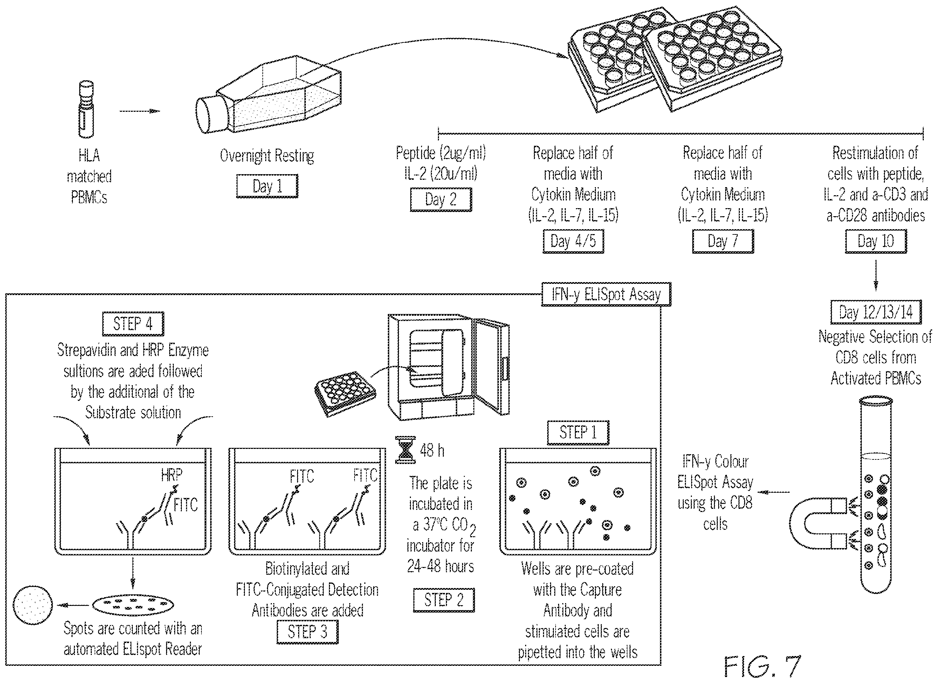

Next, the immunogenic peptides were further screened for effectiveness as vaccine candidates in accordance with the scheme illustrated in **FIG. 7****.** First, an *in vitro* Enzyme-Linked Immunospot (ELISpot) assay was established where the CD8 T cell responses were assessed after a long-term culture of peripheral blood mononuclear cells (PBMCs) from an HLA-matched healthy donor. The response was assessed through the enumeration of antigen specific IFN-γ secreting T cells.

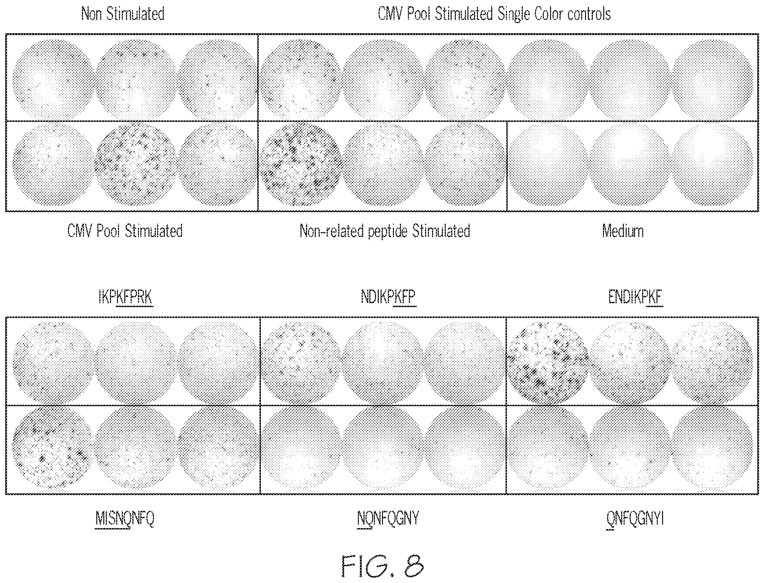

The results illustrated in **FIGS. 8-9** demonstrate the suitability of the established PBMC-based system for the *in vitro* validation of the neoantigen peptides selected through MHCnuggets.

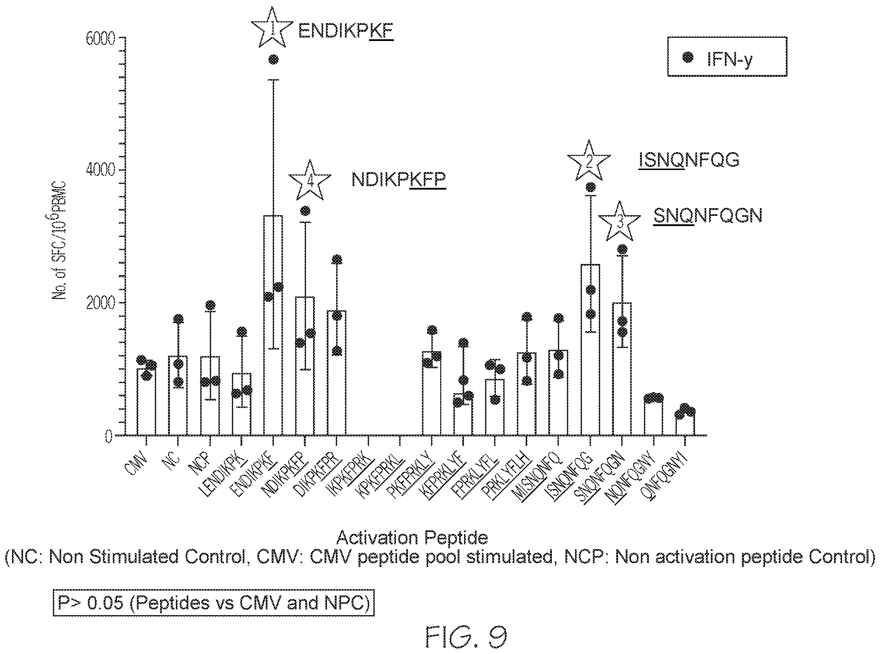

**FIG. 8** illustrates images of the ELISpot plate with control wells and sample wells. **FIG. 9** illustrates human IFNγ dual colour ELISpot Assay for predicted immunogenic neoantigenic peptides of NSF-LRRC37A2. ENDIKPKF, NDIKPKFP, ISNQNFQG, and SNQNFQGN neopeptides were recognized as promising candidates through the ELISpot Assay.

### Example 2. mRNA Vaccines for the Treatment and Prevention of Cancer

This Example illustrates mRNA design for chimeric fusion protein candidates. **FIG. 10** illustrates a synthetic mRNA design following the structure of eukaryotic mRNA. The mRNA design was modeled after a eukaryotic mRNA template. Peptide cassettes were designed to include neoantigentic regions of proteins derived from chimeric RNAs, with 5'- and 3'- region cassettes and spacers on each side experimentally determined by *in-vitro expression.* **Table 2** lists the sequences of the different cassettes of the mRNA vaccine.

**Table 2. Sequences of the different cassettes of the mRNA vaccine design in FIG. 10.**

| **Cassette** | **Sequence** |
|---|---|
| 5' Region Cassette 1 | ACATTTGCTTCTGACACAACTGTGTTCACTAGCAACCTCAAACAGACACC |
| 5' Region Cassette 2 | AGCACCACGGCAGCAGGAGGTTTCGGCTAAGTTGGAGGTACTGGCCACGACTGCAT GCCCGCGCCCGCCAGGTGATACCTCCGCCGGTGACCCAGGGGCTCTGCGACACAAG GAGTCTGCATGTCTAAGTGCTAGAC |
| 5' Region Cassette 3 | ACCGCCGAGACCGCGTCCGCCCCGCGAGCACAGAGCCTCGCCTTTGCCGATCCGCCG CCCGTCCACACCCGCCGCCAGCTCACC |
| 5' Region Cassette 4 | CTTCCTTTCCAACTTGGACGCTGCAGA |
| 5' Spacer | CCACC |
| Peptide Cassette | TGGAGAATGATATCAAACCAAAATTTCCAAGGAAACTATAT |
| 3' Spacer Cassette 1 | GCTCGCTTTCTTGCTGTCCAATTTCTATTAAAGGTTCCTTTGTTCCCTAAGTCCAACTA CTAAACTGGGGGATATTATGAAGGGCCTTGAGCATCTGGATTCTGCCTAATAAAAAA CATTTATTTTCATTGCA |
| 3' Spacer Cassette 2 | ATGAACTCAATCTAAATTAAAAAAGAAAGAAATTTGAAAAAACTTTCTCTTTGCCAT TTCTTCTTCTTCTTTTTTAACTGAAAGCTGAATCCTTCCATTTCTTCTGCACATCTACT TGCTTAAATTGTGGGCAAAAGAGAAAAAGAAGGATTGATCAGAGCATTGTGCAATA CAGTTTCATTAACTCCTTCCCCCGCTCCCCCAAAAATTTGAATTTTTTTTCAACACTC TTACACCTGTTATGGAAAATGTCAACCTTTGTAAGAAAACCAAAATAAAAATTGAAA AATAAAAACCATAAACATTTGCACCACTTGTGGCTTTTGAATATCTTCCACAGAGGG AAGTTTAAAACCCAAACTTCCAAAGGTTTAAACTACCTCAAAACACTTTCCCATGAG TGTGATCCACATTGTTAGGTGCTGACCTAGACAGAGATGAACTGAGGTCCTTGTTTT GTTTTGTTCATAATACAAAGGTGCTAATTAATAGTATTTCAGATACTTGAAGAATGTT GATGGTGCTAGAAGAATTTGAGAAGAAATACTCCTGTATTGAGTTGTATCGTGTGGT GTATTTTTTAAAAAATTTGATTTAGCATTCATATTTTCCATCTTATTCCCAATTAAAA GTATGCAGATTATTTGCCCAAATCTTCTTCAGATTCAGCATTTGTTCTTTGCCAGTCT CATTTTCATCTTCTTCCATGGTTCCACAGAAGCTTTGTTTCTTGGGCAAGCAGAAAAA TTAAATTGTACCTATTTTGTATATGTGAGATGTTTAAATAAATTGTGAAAAAAATGA AATAAAGCATGTTTGGTTTTCCAAAAGAACATAT |
| 3' Spacer Cassette 3 | GCGGACTATGACTTAGTTGCGTTACACCCTTTCTTGACAAAACCTAACTTGCGCAGA AAACAAGATGAGATTGGCATGGCTTTATTTGTTTTTTTGTTTTGTTTTGGTTTTTTTT TTTTTTTGGCTTGACTCAGGATTTAAAAACTGGAACGGTGAAGGTGACAGCAGTCG GTTGGAGCGAGCATCCCCCAAAGTTCACAATGTGGCCGAGGACTTTGATTGCACATT GTTGTTTTTTTAATAGTCATTCCAAATATGAGATGCGTTGTTACAGGAAGTCCCTTGC CATCCTAAAAGCCACCCCACTTCTCTCTAAGGAGAATGGCCCAGTCCTCTCCCAAGT CCACACAGGGGAGGTGATAGCATTGCTTTCGTGTAAATTATGTAATGCAAAATTTTT TTAATCTTCGCCTTAATACTTTTTTATTTTGTTTTATTTTGAATGATGAGCCTTCGTGC |
| | CCCCCCTTCCCCCTTTTTGTCCCCCAACTTGAGATGTATGAAGGCTTTGGTCTCCCT GGGAGTGGGTGGAGGCAGCCAGGGCTTACCTGTACACTGACTTGAGACCAGTTGAA TAAAAGTGCACACCTTAAAAATGA |
| 3' Spacer Cassette 4 | AGCCATTTAAATTCATTAGAAAAATGTCCTTACCTCTTAAAATGTGAATTCATCTGTT AAGCTAGGGGTGACACACGTCATTGTACCCTTTTTAAATTGTTGGTGTGGAAGATG CTAAAGAATGCAAAACTGATCCATATCTGGGATGTAAAAAGGTTGTGGAAAATAGA ATGCCCAGACCCGTCTACAAAAGGTTTTTAGAGTTGAAATATGAAATGTGATGTGGG TATGGAAATTGACTGTTACTTCCTTTACAGATCTACAGACAGTCAATGTGGATGAGA ACTAATCGCTGATCGTCAGATCAAATAAAGTTATAAAATTGC |
| 3' Region Cassette | A30(GCATATGACT)A70 |

## Claims

1. A composition comprising:
at least one immunogenic peptide, a nucleotide sequence that expresses the immunogenic peptide, or combinations thereof,
wherein the immunogenic peptide comprises one or more peptides selected from the group consisting of ENDIKPKF (SEQ ID NO: 6), NDIKPKFP (SEQ ID NO: 7), ISNQNFQG (SEQ ID NO: 16), SNQNFQGN (SEQ ID NO: 17), analogs thereof, homologs thereof, or combinations thereof,
wherein the analog or a homolog is at least 80% identical to any of the immunogenic peptides.

2. The composition of claim 1, wherein the immunogenic peptides are expressed at a junction region between N-ethylmaleimide sensitive factor, vesicle fusing ATPase, transcript variant 1 (NSF) and Leucine Rich Repeat Containing 37 Member A3 (LLRC37A3) (NSF-LRRC37A3).

3. The composition of claim 1, wherein the immunogenic peptide is capable of eliciting an immune response through binding to human leukocyte antigen (HLA) systems or complexes, and wherein the HLA systems or complexes comprise major histocompatibility complex (MHC) proteins, MHC class I (MHC I) proteins, MHC class II (MHC II) proteins, or combinations thereof.

4. The composition of claim 1, wherein the nucleotide sequence is in the form of a DNA sequence.

5. The composition of claim 1, wherein the nucleotide sequence is in the form of an RNA sequence.

6. The composition of claim 1, wherein the nucleotide sequence comprises a mRNA expression cassette, wherein the expression cassette comprises a peptide cassette that contains the nucleotide sequence expressing the immunogenic peptide, a 5' cassette region upstream the peptide cassette, a spacer region between the 5' cassette region and the peptide cassette, a 3' cassette region downstream the peptide cassette, and a spacer region between the peptide cassette and the 3' cassette region, wherein at least one of the 5' cassette region and 3' cassette region is designed to optimize the expression of the immunogenic peptide.

7. The composition of claim 1, wherein the composition further comprises an immune adjuvant, wherein the immune adjuvant is selected from the group consisting of analgesic adjuvants, inorganic compounds, mineral oil, bacterial products, non-bacterial inorganics, delivery systems, plant-based products, cytokines, food-based oil, or combinations thereof.

8. The composition of any preceding claim for use in treating or preventing breast cancer in a subject. upon administration of the composition to the subject.

9. The composition for use of claim 8, wherein the subject is a human being.

10. The composition for use of claims 8 or 9, wherein the administering comprises administering the immunogenic peptide.

11. The composition for use of claims 8 or 9, wherein the administering comprises administering the nucleotide sequence expressing the immunogenic peptide.

## Patentansprüche

1. Zusammensetzung, umfassend:
mindestens ein immunogenes Peptid, eine das immunogene Peptid exprimierende Nukleotidsequenz oder Kombinationen davon,
wobei das immunogene Peptid ein oder mehrere Peptide umfasst, die ausgewählt sind aus der Gruppe bestehend aus ENDIKPKF (SEQ ID NO: 6), NDIKPKFP (SEQ ID NO: 7), ISNQNFQG (SEQ ID NO: 16), SNQNFQGN (SEQ ID NO: 17), Analoga davon, Homologen davon oder Kombinationen davon, wobei das Analogon oder ein Homolog mindestens 80 % identisch mit einem beliebigen der immunogenen Peptide ist.

2. Zusammensetzung nach Anspruch 1, wobei die immunogenen Peptide an einer Übergangsregion zwischen N-Ethylmaleimid-sensitivem Faktor, Vesikel-fusionierender ATPase, Transkriptvariante 1 (NSF) und Leucine Rich Repeat Containing 37 Member A3 (LLRC37A3) (NSF-LRRC37A3) exprimiert werden.

3. Zusammensetzung nach Anspruch 1, wobei das immunogene Peptid in der Lage ist, eine Immunantwort durch Bindung an Systeme oder Komplexe des menschlichen Leukozytenantigens (HLA) hervorzurufen und wobei die HLA-Systeme oder -Komplexe Proteine des Haupthistokompatibilitätskomplexes (MHC), Proteine der MHC-Klasse I (MHC I), Proteine der MHC-Klasse II (MHC II) oder Kombinationen davon umfassen.

4. Zusammensetzung nach Anspruch 1, wobei die Nukleotidsequenz in Form einer DNA-Sequenz vorliegt.

5. Zusammensetzung nach Anspruch 1, wobei die Nukleotidsequenz in Form einer RNA-Sequenz vorliegt.

6. Zusammensetzung nach Anspruch 1, wobei die Nukleotidsequenz eine mRNA-Expressionskassette umfasst, wobei die Expressionskassette eine Peptidkassette, welche die das immunogene Peptid exprimierende Nukleotidsequenz enthält, eine 5'-Kassettenregion stromaufwärts der Peptidkassette, eine Spacer-Region zwischen der 5'-Kassettenregion und der Peptidkassette, eine 3'-Kassettenregion stromabwärts der Peptidkassette und eine Spacer-Region zwischen der Peptidkassette und der 3'-Kassettenregion umfasst, wobei mindestens eine von der 5'-Kassettenregion und der 3'-Kassettenregion dazu ausgelegt ist, die Expression des immunogenen Peptids zu optimieren.

7. Zusammensetzung nach Anspruch 1, wobei die Zusammensetzung ferner ein Immunadjuvans umfasst, wobei das Immunadjuvans ausgewählt ist aus der Gruppe bestehend aus analgetischen Adjuvanzien, anorganischen Verbindungen, Mineralöl, bakteriellen Produkten, nicht-bakteriellen Anorganika, Abgabesystemen, pflanznbasierten Produkten, Zytokinen, Speiseöl oder Kombinationen davon.

8. Zusammensetzung nach einem vorhergehenden Anspruch zur Verwendung bei der Behandlung oder Prävention von Brustkrebs bei einem Subjekt bei Verabreichung der Zusammensetzung an das Subjekt.

9. Zusammensetzung zur Verwendung nach Anspruch 8, wobei das Subjekt ein Mensch ist.

10. Zusammensetzung zur Verwendung nach Anspruch 8 oder 9, wobei die Verabreichung die Verabreichung des immunogenen Peptids umfasst.

11. Zusammensetzung zur Verwendung nach Anspruch 8 oder 9, wobei die Verabreichung die Verabreichung der das immunogene Peptid exprimierenden Nukleotidsequenz umfasst.

## Revendications

1. Composition comprenant :
au moins un peptide immunogène, une séquence nucléotidique qui exprime le peptide immunogène, ou des combinaisons de ceux-ci,
ledit peptide immunogène comprenant un ou plusieurs peptides choisis dans le groupe constitué de ENDIKPKF (SEQ ID N° : 6), NDIKPKFP (SEQ ID N° : 7), ISNQNFQG (SEQ ID N° : 16), SNQNFQGN (SEQ ID N° : 17), des analogues de ceux-ci, des homologues de ceux-ci ou des combinaisons de ceux-ci,
l'analogue ou un homologue étant identique à au moins 80 % à l'un quelconque des peptides immunogènes.

2. Composition de la revendication 1, dans laquelle les peptides immunogènes sont exprimés au niveau d'une région de jonction entre le facteur sensible au N-éthylmaléimide, la vésicule fusionnant l'ATPase, le variant de transcrit 1 (NSF) et la répétition riche en leucine contenant 37 éléments A3 (LLRC37A3) (NSF-LRRC37A3).

3. Composition de la revendication 1, dans laquelle le peptide immunogène est capable de provoquer une réponse immunitaire par liaison à des systèmes ou complexes de l'antigène leucocytaire humain (HLA), et dans laquelle les systèmes ou complexes HLA comprennent des protéines du complexe majeur d'histocompatibilité (CMH), des protéines du CMH de classe I (CMH I), des protéines du CMH de classe II (CMH II) ou des combinaisons de celles-ci.

4. Composition de la revendication 1, dans laquelle la séquence nucléotidique se présente sous la forme d'une séquence d'ADN.

5. Composition de la revendication 1, dans laquelle la séquence nucléotidique se présente sous la forme d'une séquence d'ARN.

6. Composition de la revendication 1, dans laquelle la séquence nucléotidique comprend une cassette d'expression d'ARNm, ladite cassette d'expression comprenant une cassette peptidique qui contient la séquence nucléotidique exprimant le peptide immunogène, une région de cassette 5' en amont de la cassette peptidique, une région espaceur entre la région de cassette 5' et la cassette peptidique, une région de cassette 3'en aval de la cassette peptidique, et une région espaceur entre la cassette peptidique et la région de cassette 3', au moins l'une desdites régions de cassette 5'et de cassette 3' étant conçue pour optimiser l'expression du peptide immunogène.

7. Composition de la revendication 1, ladite composition comprenant en outre un adjuvant immunitaire, ledit adjuvant immunitaire étant choisi dans le groupe constitué des adjuvants analgésiques, des composés inorganiques, de l'huile minérale, des produits bactériens, des produits inorganiques non bactériens, des systèmes d'administration, des produits à base de plantes, des cytokines, de l'huile alimentaire ou des combinaisons de ceux-ci.

8. Composition de l'une quelconque des revendications précédentes, destinée à être utilisée dans le traitement ou la prévention du cancer du sein chez un sujet, lors de l'administration de la composition au sujet.

9. Composition destinée à être utilisée selon la revendication 8, ledit sujet étant un être humain.

10. Composition destinée à être utilisée selon la revendication 8 ou 9, ladite administration comprenant l'administration du peptide immunogène.

11. Composition destinée à être utilisée selon la revendication 8 ou 9, ladite administration comprenant l'administration de la séquence nucléotidique exprimant le peptide immunogène.
